# EUROPEAN PATENT APPLICATION

(11) **EP 4 454 847 A1**
(43) Date of publication of application: **30.10.2024**
(21) Application number: 22909877.7
(22) Date of filing: 16.12.2022
(51) Int. Cl.: B27N 3/04, C12N 1/14

(54) **LEATHER-LIKE BIOLOGICAL COMPOSITE MATERIAL, PREPARATION METHOD THEREFOR AND USE THEREOF**

(30) Priority: 21.12.2021 CN 202111570984
(71) Applicant: Shenzhen Institute of Advanced Technology Chinese Academy of Sciences, Shenzhen, Guangdong 518055 (CN)
(72) Inventor: LIU, Chenli, Shenzhen, Guangdong 518055 (CN); CHENG, Hao, Shenzhen, Guangdong 518055 (CN)
(74) Representative: Michalski Hüttermann & Partner Patentanwälte mbB
(86) International application number: PCT/CN2022/139524
(87) International publication number: WO 2023/116563

(57) **Abstract**

The present invention provides a method for preparing a leather-like biocomposite comprises: culturing a culture mixture comprising a fungal strain and a nutrient substrate in a culture device; flat pressing a plate against the upper surface of mycelium in one direction and continuing the culture; removing the plate and closely attaching a lattice-like membrane on the upper surface of the mycelium and continuing the culture; flat pressing the plate against the upper surface of mycelium in one direction and continuing the culture until a mycelium-membrane complex is formed where the mycelium completely covers the lattice-like membrane; repeating the foregoing steps at least once; and separating the complex from the nutrient substrate and post-treating the complex. The present invention also provides a leather-like biocomposite prepared by the method of the present invention, and use thereof for preparing a textile product. The method of the present invention is economical and friendly environmental, and the leather-like biocomposite obtained by the method exhibits a uniform texture, a soft and fine handfeel, and high strength mechanical properties.

## Description

The present application claims priority to Chinese patent application CN202111570984.3, filed on December 21, 2021, entitled **"LEATHER-LIKE BIOLOGICAL COMPOSITE MATERIAL, PREPARATION METHOD THEREFOR AND USE THEREOF",** the entire content of which is incorporated herein by reference.

### Technical Field

The present invention relates to a leather-like biocomposite, preparation method therefor and use thereof. Particularly, the present invention relates to a method for preparing a leather-like biocomposite made from a fungal mycelium, a leather-like biocomposite prepared by the method, and use of the leather-like biological composite for preparing a textile product.

### Background

Traditionally, leather comes from animals. Producing animal leather is cumbersome, and raising livestock requires a large amount of resource and may take years, which often introduces production crises and price volatility. In addition, such production method also has potential ethical issues.

Traditional leather also comes from synthetic materials. Synthetic materials are often derived from non-renewable resources or limited natural resources, which is not sustainable and even generates significant waste discharges. The leather waste after use is often harmful to the environment and nature. Therefore, it has been in the spotlight to produce a new green, sustainable, naturally degradable, and non-polluting material without being limited by time and space constraints.

Mycelium, one of the best decomposers in nature, exists in part of the life cycle of certain fungi. It is capable of attaching to culture media based on biowaste and agricultural waste, digesting them for promoting mycelium growth, and self-assembling into an intricate mycelium network. In contrast to livestock raising, mycelium can grow in a few days. In addition, unlike leather made from synthetic fibers, the material is made from mycelium, which does not need to be extracted from petroleum, making it environment-friendly. At the same time, it can meet more aesthetic and functional needs of consumers. Therefore, it is an ethically and environmentally responsible biomaterial.

### Summary

One of the technical problems to be solved by the present invention is to provide a leather-like biocomposite that can replace animal leather. One of the technical problems to be solved by the present invention is to improve the hand feel, texture and appearance of the resulting leather-like biocomposite. One of the technical problems to be solved by the present invention is to improve the mechanical properties of the resulting leather-like biocomposite, including elongation at break, breaking strength, and tear strength. One of the technical problems to be solved by the present invention is to provide a method for preparing the leather-like biocomposite with the above improvements.

In one aspect, the present invention provides a method for preparing a leather-like biocomposite, comprising the following steps:
i). placing a culture mixture comprising a fungal strain and a nutrient substrate in a culture device and culturing the fungal strain until mycelium of the fungal strain grows to a height of 0.5-1 cm and distributes evenly in the entire nutrient substrate;
ii). flat pressing an unperforated plate against the upper surface of the mycelium in one direction and continuing culturing until the mycelium attaching to the plate grows to a thickness of 0.1-5 mm and the formed mycelium completely covers the entire nutrient substrate, forming a mycelial membrane structure on the surface thereof;
iii). removing the unperforated plate, attaching a lattice-like membrane onto the upper surface of the mycelium and continuing culturing until the mycelium completely penetrates the lattice-like membrane and grows uniformly to a height of 0.5-1 cm, without forming a mycelial membrane structure;
iv). flat pressing the unperforated plate against the upper surface of the mycelium in one direction and continuing culturing until the mycelium grows to a thickness of 1-5 mm and a mycelium-membrane complex is formed where the mycelium completely covers the lattice-like membrane, forming a mycelial membrane structure again on the surface;
v). repeating the preceding steps iii) and iv) at least once; and
vi). separating the complex from the nutrient substrate and post-treating the complex to obtain a leather-like biocomposite.

In another aspect, the present invention provides a leather-like biocomposite prepared by the method of the present invention.

In yet another aspect, the present invention provides use of the leather-like biocomposite of the invention for preparing a textile product.

The method for preparing the leather-like biocomposite of the present invention does not need to use a material derived from animals or chemical processes, so it is economical and environment-friendly, and has wide applications in various industrial and living scenarios. More importantly, taking advantage of the capability of the fungal mycelium to self-assemble into intricate mycelium networks, the method of the present invention is used to prepare biodegradable biomaterials with excellent mechanical properties. The leather-like biocomposite obtained by the preparation method of the present invention exhibits a uniform texture, a soft and fine hand feel, and high strength mechanical properties.

### Brief Description of Drawings

FIG. 1 shows schematic flowcharts of a method for preparing a leather-like biocomposite of the present invention:
   FIG. 1A shows a schematic flowchart of a method for preparing a leather-like biocomposite of the present invention; and
   FIG. 1B shows another schematic flowchart of a method for preparing a leather-like biocomposite according to the present invention.
FIG. 2 shows a schematic diagram of a culture device used for the method for preparing the leather-like biocomposite of the present invention.
FIG. 3 shows physical diagrams of culturing states corresponding to part of the steps in the method for preparing the leather-like biocomposite in Example 1 of the present invention:
   FIG. 3A shows a physical diagram of a culture mixture of a fungal strain and a nutrient substrate;
   FIG. 3B shows a physical diagram of mycelium of the strain that grew and filled the entire nutrient substrate after a period of culturing;
   FIG. 3C shows a physical diagram of mycelium that was stringently controlled to grow to a height of 0.6 cm and distributed evenly in the foregoing step 5;
   FIG. 3D shows a physical diagram of the plate used for flat pressing in the foregoing step 6;
   FIG. 3E shows a physical diagram of the final state where the upper surface of the mycelium is flat pressed in one direction in the foregoing step 6;
   FIG. 3F shows a physical diagram of the final state in the foregoing step 6 where culturing by flat pressing using a plate was performed until the mycelium at the edge was 1.5 mm thick and the formed mycelium completely covered the entire nutrient substrate to form a mycelial membrane structure on the surface, as well as a localized magnified diagram thereof;
   FIG. 3G shows a physical diagram of the final state in the forgoing step 7 where a pure cotton gauze with an average pore size of 100 µm was closely attached to the upper surface of the mycelium;
   FIG. 3H shows a physical diagram in the foregoing step 7 where the mycelium was controlled to grow to completely penetrate the lattice-like membrane and grew to a height of 0.7 cm and was uniformly distributed, without forming a membrane structure;
   FIG. 3I shows a physical diagram in the foregoing step 8 where a mycelium-membrane complex was finally formed where the mycelium completely covered the lattice-like membrane, forming a mycelial membrane structure on the surface;
   FIG. 3J shows a physical diagram of the final leather-like biocomposite resulting from the forgoing processing step 11.
FIG. 4 shows physical comparison of the mycelial membrane structure obtained by the method of examples of the present invention to a non-mycelial membrane structure.
FIG. 5 shows physical diagrams of the structures of mycelium layers obtained by rolling and plate pressing, respectively;
   FIG. 5A shows a physical diagram of the structure of the mycelium layer obtained by a single light rolling;
   FIG. 5B shows a physical diagram of the structure of the mycelium layer obtained by two times of moderate rolling;
   FIG. 5C shows a physical diagram of the structure of the mycelium layer obtained by several times of heavy rolling; and
   FIG. 5D shows physical diagrams of the structure of the mycelium layer obtained by plate pressing of the present invention.
FIG. 6 shows a physical diagram of a leather-like biocomposite obtained by the method of examples of the present invention and post-treating, which was sent to CTI for mechanical performance testing.
FIG. 7 shows a physical diagram of a leather-like biocomposite obtained by the method of examples of the present invention and post-treating, and a hat made therefrom.

### Detailed Description

The exemplary embodiments are described in detail below with reference to the accompanying drawings. The drawings are used to illustrate the present invention and are not intended to limit it.

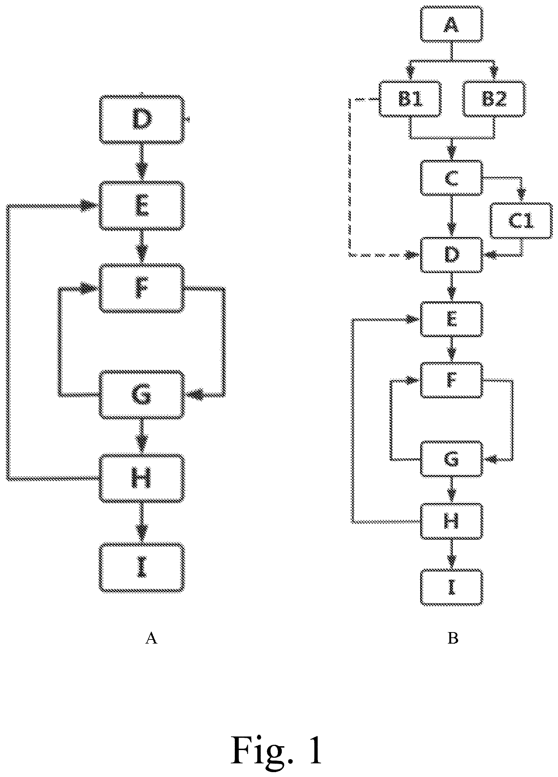

In one aspect, the present invention provides a method for preparing a leather-like biocomposite. FIG. 1A shows a schematic flowchart of the method for preparing a leather-like biocomposite of the present invention.

As shown in FIG. 1A, the method for preparing a leather-like biocomposite comprises: placing a suitable amount of culture mixture comprising a fungal strain and a nutrient substrate in a culture device and culturing it until the mycelium on the surface of the nutrient substrate grows to a height of 0.5-1 cm and distributes evenly (step D); flat pressing a plate against the upper surface of the mycelium in one direction and culturing until the mycelium attaching to the plate grows to a thickness of 0.1-5 mm and the formed mycelium completely covers the entire nutrient substrate, forming a mycelial membrane structure on the surface thereof (step E); removing the plate, closely attaching a lattice-like membrane on the upper surface of the mycelium and continuing culturing until the mycelium completely penetrates the lattice-like membrane and grows to a height of 0.5-1 cm and distributes evenly, without forming a mycelial membrane structure (step F); flat pressing a plate against the upper surface of the mycelium again in one direction and continuing culturing until the mycelium grows to a thickness of 1-5 mm and a mycelium-membrane complex is formed where the mycelium completely covers the lattice-like membrane, forming a mycelial membrane structure again on the surface (step G); repeating the processes of step F to step G at least once; separating the mycelium-membrane complex (containing a lattice-like membrane) obtained in step G from the nutrient substrate attached to its lower part (step H) at the upper lattice-like membrane; optionally, placing the separated nutrient substrate in step H in the culture device since the nutrient substrate has culture strains penetrated therein in the preceding culture processes, and then repeating the processes of step D to step H;
post-treating the separated mycelium-membrane complex obtained in step H (step I) to prepare a leather-like biocomposite.

In one embodiment, the present invention provides a method for preparing a leather-like biocomposite, comprising the following steps:
i). placing a culture mixture comprising a fungal strain and a nutrient substrate in a culture device and culturing the fungal strain until mycelium of the fungal strain grows to a height of 0.5-1 cm and distributes evenly in the entire nutrient substrate;
ii). flat pressing an unperforated plate against the upper surface of the mycelium in one direction and continuing culturing until the mycelium attaching to the plate grows to a thickness of 0.1-5 mm and the formed mycelium completely covers the entire nutrient substrate, forming a mycelial membrane structure on the surface thereof;
iii). removing the unperforated plate, closely attaching a lattice-like membrane on the upper surface of the mycelium and continue culturing until the mycelium completely penetrates the lattice-like membrane and grows to a height of 0.5-1 cm and distributes evenly, without forming a mycelial membrane structure;
iv). flat pressing the unperforated plate on the upper surface of the mycelium in one direction and continuing culturing until the mycelium grows to a thickness of 1-5 mm and a mycelium-membrane complex is formed where the mycelium completely covers the lattice-like membrane, forming a mycelial membrane structure again on the surface;
v). repeating the preceding steps iii) and iv) at least once; and
vi). separating the complex from the nutrient substrate and post-treating the complex to obtain a leather-like biocomposite.

In the present invention, the fungal strain in step i) can be cultured through the following steps, including: placing a certain amount of a culture mixture comprising the fungal strain and a nutrient substrate in a culture device, with a volume ratio of the mixture in the culture device not exceeding 60%, compacting and flat pressing the mixture in the culture device, and kept the mixture standstill for culturing in darkness at a temperature of 25-35 °C, a concentration of carbon dioxide of 3-7%, an oxygen concentration ≤20% and a relative humidity ≥40% for 3-7 days, e.g., 3 days, 4 days, 5 days, 6 days or 7 days.

In one embodiment, in step i), the fungal strain is cultured under the following conditions, until the mycelium of the fungal strain grows and fills the entire nutrient substrate. The nutrient substrate is a nutrient substrate comprising lignin or a derivative thereof. The volume ratio of the culturing mixture in the culture device is up to 60%. The culture duration is 3-7 days. The temperature is 25-35°C. The culture mixture is kept standstill in darkness. The concentration of carbon dioxide is 3-7%; the concentration of oxygen is ≤20%; and a relative humidity is ≥ 40%. In one embodiment, the nutrient substrate is selected from one or more of the following substances: straw, rice hulls, fuelwood, bark, peanut hulls, corn cobs, twig firewood, wrapper, wood chips and a mixture thereof.

In one embodiment, in step i), the mycelium grows to a height selected from 0.5 cm, 0.6 cm, 0.7 cm, 0.8 cm, 0.9 cm, 1.0 cm and a range consisting of any two of the preceding values. In one embodiment, in step i), the mycelium grows to a height of 0.5 cm, 0.6 cm, or 0.7 cm.

Controlling the growth of the mycelium in steps ii)-iv), such as the growth height and the densification state allows the mycelium to self-assemble into an intricate mycelium network and completely cover and twist with the lattice-like membrane to form a robust, biodegradable material. The lattice-like membrane in the mycelium-membrane complex can provide the necessary structural support for the complex, further enhancing the mechanical properties such as breaking strength of the complex.

In step ii) of the method of the present invention, the mycelium is allowed to grow for a period of time while being flat pressed by an unperforated plate until the mycelium attaching to the plate grows to a thickness of 0.1-5 mm and the formed mycelium completely covers the entire nutrient substrate, forming a mycelial membrane structure on the surface. In one embodiment, an unperforated plate was flat pressed on the upper surface of the mycelium in one direction and the culture continued until the mycelium attaching to the plate grew to a thickness of 0.1-5 mm and the formed mycelium completely covered the entire nutrient substrate, forming a mycelial membrane structure on the surface thereof. Flat pressing a plate against it for culturing blocked the mycelium growth upwards, making it to attach the plate and grow laterally, and intricately twisted into a membrane plane. This layer of mycelial membrane facilitates the separation of the lower nutrient substrate from the upper mycelium composite in subsequent steps. In addition, the composite growing on this layer of mycelial membrane structure can have a uniformly fine and smooth surface at both sides, which allows for subsequent industrialization of the material without the need for further treatment/processing.

In one embodiment, in step ii) of the method of the present invention, the mycelium grows to a thickness selected from a group consisting of: 0.1 mm, 0.2 mm, 0.3 mm, 0.4 mm, 0.5 mm, 0.6 mm, 0.7 mm, 0.8 mm, 0.9 mm, 1 mm, 1.1 mm, 1.2 mm, 1.3 mm, 1.4 mm, 1.5 mm, 1.6 mm, 1.7 mm, 1.8 mm, 1.9 mm, 2 mm, 2.1 mm, 2.2 mm, 2.3 mm, 2.4 mm, 2.5 mm, 2.6 mm, 2.7 mm, 2.8 mm, 2.9 mm, 3 mm, 3.1 mm, 3.2 mm, 3.3 mm, 3.4 mm, 3.5 mm, 3.6 mm, 3.7 mm, 3.8 mm, 3.9 mm, 4 mm, 4.1 mm, 4.2 mm, 4.3 mm, 4.4 mm, 4.5 mm, 4.6 mm, 4.7 mm, 4.8 mm, 4.9 mm, 5mm, and a range between any two of the preceding numbers. In one embodiment, in step ii) of the method of the present invention, the mycelium grows to a thickness from 1 mm to 2 mm. In one embodiment, in step ii) of the method of the present invention, the mycelium grows to a thickness of 1 mm, 1.1 mm, 1.2 mm, 1.3 mm, 1.4 mm, 1.5 mm, 1.6 mm, 1.7 mm, 1.8 mm, 1.9 mm, or 2 mm.

In step iii) of the method of the present invention, the mycelium growth needs to be controlled to completely penetrate the lattice-like membrane and grow to a height of 0.5-1 cm and distributes evenly, where the upright or branched mycelium is in a growing state in which the interstices are not connected in appearance. In one embodiment, the unperforated plate is removed, a lattice-like membrane is closely attached onto the upper surface of the mycelium and the culture is continued until the mycelium completely penetrates the lattice-like membrane and grows uniformly distributed to a height of 0.5-1 cm and distributes evenly, where the upright or branched mycelium is in a growing state in which the interstices are not connected in appearance, without forming a mycelial membrane structure.

In one embodiment, in step iii) of the method of the present invention, the height of the mycelial growth is selected from 0.5 cm, 0.6 cm, 0.7 cm, 0.8 cm, 0.9 cm, 1 cm and a range consisting of any two of the preceding values. In one embodiment, in step iii) of the method of the present invention, the mycelium growth needs to be controlled to a height of 0.5 cm to 0.8 cm. In one embodiment, in step iii) of the method of the present invention, the mycelium growth needs to be controlled to a height of 0.5 cm, 0.6 cm, 0.7 cm to 0.8 cm.

In step iv) of the method of the present invention, the mycelium is again allowed to grow for a period of time while being flat pressed by an unperforated plate until the mycelium grows to a thickness of 1-5 mm and reaches a state where the mycelium completely covers the lattice-like membrane without visible interstices or uneven distribution, and forms an overall fine and smooth surface. In one embodiment, an unperforated plate is flat pressed against the upper surface of the mycelium in one direction and the culture is continued until the mycelium grows to a thickness of 1-5 mm and reaches a growing state where the mycelium completely covers the lattice-like membrane without visible interstices or uneven distribution, and forms an overall fine and smooth surface, forming a mycelial membrane structure again on the surface. The formation of the mycelial membrane structure again increases the mechanical strength of the mycelium-membrane complex, and enhances the flatness and smooth hand feel.

In one embodiment, in step iv) of the method of the present invention, the mycelium grew to a thickness selected from the group consisting of: 1 mm, 1.1 mm, 1.2 mm, 1.3 mm, 1.4 mm, 1.5 mm, 1.6 mm, 1.7 mm, 1.8 mm, 1.9 mm, 2 mm, 2.1 mm, 2.2 mm, 2.3 mm, 2.4 mm, 2.5 mm, 2.6 mm, 2.7 mm, 2.8 mm, 2.9 mm, 3 mm, 3.1 mm, 3.2 mm, 3.3 mm, 3.4 mm, 3.5 mm, 3.6 mm, 3.7 mm, 3.8 mm, 3.9 mm, 4 mm, 4.1 mm, 4.2 mm, 4.3 mm, 4.4 mm, 4.5 mm, 4.6 mm, 4.7 mm, 4.8 mm, 4.9 mm, 5mm, and a range between any two of the preceding numbers. In one embodiment, in step iv) of the method of the present invention, the mycelium grows to a thickness from 1 mm to 2 mm. In one embodiment, in step iv) of the method of the present invention, the mycelium grows to a thickness of 1 mm, 1.1 mm, 1.2 mm, 1.3 mm, 1.4 mm, 1.5 mm, 1.6 mm, 1.7 mm, 1.8 mm, 1.9 mm, or 2 mm.

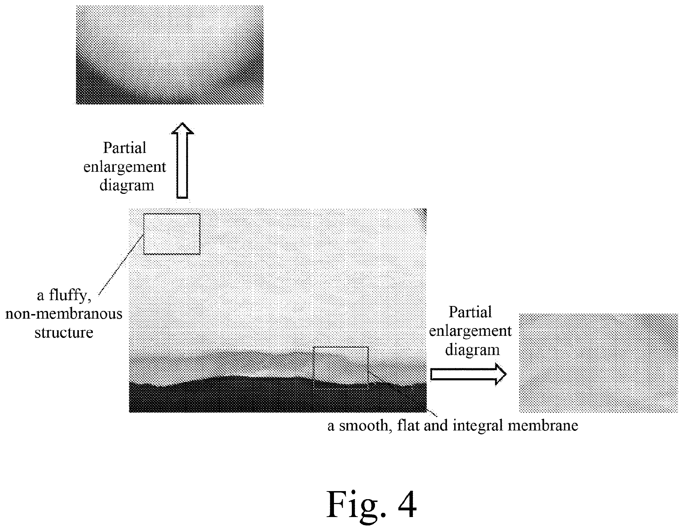

In the present invention, the term "mycelial membrane structure" means that the mycelium has an integral and intact a membrane-like structure and a smooth and flat upper surface. On contrary, if the mycelium is dispersed or intensively arranged without an integral structure, exhibiting a fluffy appearance, then the mycelium in this state is referred to as a non-membranous mycelial structure. See FIG. 4 for a physical diagram of the mycelial membrane structure.

In the present invention, it is unexpected that the operation of flat plate pressing allows the final monolayer mycelium-membrane complex to have excellent texture, hand feel, appearance, and mechanical properties, as compared to rolling, an easy-to-operate method. Without being bound by any theory, the inventor believes that employing the flat-platen pressing operation during the mycelium growth can control the mycelium growth on one plane, regardless of differences in growth direction and status of each hypha, which is critical for the formation of the mycelial membrane structure. Furthermore, the flat-platen pressing operation can significantly increase the mycelium density in the mycelium-membrane complex and the thickness of the entire mycelium-membrane complex, with a finer mycelial membrane structure formed on the surface, enabling the final monolayer mycelium-membrane complex to have excellent mechanical properties.

In one embodiment, in steps ii) and iv), the unperforated plate may be flat pressed in a randomized direction against the upper surface of the mycelium. In one embodiment, in steps ii) and iv), the unperforated plate may be flat pressed against the upper surface of the mycelium in such a direction that the mycelium does not cross with each other after being pressed down. The flat pressing method of the present invention can control the mycelium to grow on one plane and twist into a uniform state, regardless of differences in growth direction and status of each hypha.

In one embodiment, in steps ii) and iv), the unperforated plate is flat pressed against the upper surface of the mycelium in the same direction. By flat pressing the unperforated plate against the upper surface of the mycelium in the same direction, the resulting mycelium-membrane complex exhibits excellent mechanical properties, such as excellent elongation at break, breaking strength, and tear strength, as compared to the mycelium-membrane complex obtained by changing the orientation of the plate during flat pressing. Without being bound by any theory, the inventors believe that the way of flat pressing the unperforated plate against the upper surface of the mycelium in the same direction ensures that the mycelium has the same orientation, and the resulting mycelium-membrane complex exhibits a uniform texture, a soft and fine hand feel, and high strength mechanical properties in the same orientation as that of the mycelium.

In the present invention, the culture conditions for fungal strains in steps ii)-iv) may be the same or different. In one embodiment, in step ii), an unperforated plate is flat pressed against the upper surface of the mycelium and kept standstill for continuous culturing in darkness for 3-7 days at a temperature of 25-35 °C, a carbon dioxide concentration of 3-7%, an oxygen concentration ≤20%, and a relative humidity ≥40%. In one embodiment, in step iii), the lattice-like membrane is closely attached to the upper surface of the mycelium and kept standstill for continuous culturing in darkness for 3-7 days at a temperature of 25-35 °C, a carbon dioxide concentration of 3-7%, an oxygen concentration ≤20%, and a relative humidity ≥40%. In one embodiment, in step iv), an unperforated plate is flat pressed against the upper surface of the mycelium and kept standstill for continuous culturing in darkness for 3-7 days at a temperature of 25-35 °C, a carbon dioxide concentration of 3-7%, an oxygen concentration ≤20%, and a relative humidity ≥40%.

In the present invention, the unperforated plate may be selected as a material that is not easily adhered to and digested by the fungal hyphae. In one embodiment, the unperforated plate is a plastic plate. In one embodiment, the unperforated plate is a glass plate.

In the present invention, the lattice-like membrane may be selected from materials having a certain pore size and a certain thickness. In one embodiment, the lattice-like membrane has a thickness of 0.1 mm - 5 mm, preferably 0.5 mm - 4 mm, more preferably 0.5 mm - 3 mm, more preferably 0.5 mm - 2 mm, more preferably 0.5 mm - 1 mm, and more preferably 0.5 mm. In one embodiment, the lattice-like membrane has an average pore size of 1 µm - 5 mm, preferably 10 µm - 5 mm, more preferably 100 µm - 5 mm. The selection of a lattice-like membrane with an appropriate aperture and thickness can ensure that a sufficient number of hyphae pass through the lattice-like membrane, thereby ensuring the texture and mechanical strength of the mycelium-membrane complex.

In the present invention, the lattice-like membrane may be selected from biodegradable materials, such as fiber materials. In one embodiment, the lattice-like membrane is a fiber material. In one embodiment, the lattice-like membrane is selected from one or more of: gauze, nylon fabric, and natural fiber fabric. The fiber material is partially consumed or biodegraded by the mycelium, and the mycelium-secreted substances twist the fibers and the hyphae together to form a composite with better physical properties, thereby improving the mechanical strength of the mycelium-membrane complex.

In the present invention, the material that can be used as the nutrient substrate is a nutrient substrate comprising lignin or a derivative thereof. In one embodiment, the nutrient substrate is selected from one or more of the following substances: straw, rice hulls, fuelwood, bark, peanut hulls, corn cobs, twig firewood, wrapper, wood chips and a mixture thereof.

In the present invention, steps iii) and iv) of the present invention are repeated at least once to ensure that the mycelium-membrane complex contains at least one layer of a lattice-like membrane after final separation of the mycelium-membrane complex from the nutrient substrate. In one embodiment, the method of the present invention further comprises: in step v), repeating the preceding steps iii) and iv) several times until a complex containing a multilayer structure is formed. In one embodiment, a lattice-like membrane made from different materials may be used in each repetition of steps iii) and iv). In one embodiment, in step v), steps ii)-v) are repeated 1-5 times, for example, 1 time, 2 times, 3 times, 4 times or 5 times. In one embodiment, steps ii)-v) are repeated 1 time or 2 times.

In the present invention, the step of separating the complex from the nutrient substrate in step vi) can be carried out by separating a second layer of lattice-like membrane upwards from the nutrient substrate from its underlying mycelium (retaining the second layer of lattice-like membrane as well as the mycelium that covers around the membrane and is partially or mostly located thereon), thereby separating the mycelium-membrane complex from the nutrient substrate and obtaining a mycelium-membrane complex containing one layer of lattice-like membrane. In this separation step, a first (i.e., the bottom) layer of lattice-like membrane is wrapped in the removed nutrient substrate. By discarding the first layer of lattice-like membrane, the obtained mycelium-membrane complex containing the second layer of lattice-like membrane has a flatter surface, and exhibits a fine overall appearance and a uniform and smooth mycelium arrangement since it has the mycelium that completely covers the lattice-like membrane, thereby ensuring that the leather-like biocomposite prepared by the mycelium-membrane complex has a soft and fine look and hand feel.

In one embodiment, steps ii)-v) are repeated once, resulting in a mycelium-membrane complex comprising two layers of lattice-like membranes prior to the separation step. The lattice-like membrane covered in the mycelium-membrane complex can improve the mechanical properties of the complex. When desired, steps ii)-v) can be repeated multiple times to prepare a mycelium-membrane complex comprising multiple layers of lattice-like membranes, which can further improve the mechanical strength of the mycelium-membrane complex. In addition, mycelium-membrane complexes with different appearances and mechanical properties can be produced by using lattice membranes made of different materials and by stacking mycelium layers for different times. This further enriches the application range of the leather-like biocomposite prepared by the method of the present invention.

In one embodiment, the method of the present invention further comprises placing the nutrient substrate separated in step vi) in a culture device again for culturing, and subsequently repeating the steps ii)-v). Since the separated nutrient substrate has been infiltrated by fungi and their mycelium during the preceding culturing processes, it can be placed directly in the culture device again for culturing without the need to supplement the fungal strains again or it only needs to supplement a small amount of the fungal strains. As a result, the method of the present invention greatly saves raw materials and allows for recycling of the preparation of leather-like biocomposites in an environmentally friendly manner.

FIG. 1B shows another schematic flowchart of the method for preparing a leather-like biocomposite of the present invention. In FIG. 1B, the same reference numerals as in FIG. 1A represent the same meaning, which will not be described again.

As shown in FIG. 1B, the method for preparing a leather-like biocomposite of the present invention further comprises the step of preparing a fungal strain for use in step D. Specifically, as shown in FIG. 1B, the method for preparing a leather-like biocomposite of the present invention further comprises: preparing a mother fungal strain (step A), preparing the mother fungal strain into a secondary strain (a secondary liquid strain, corresponding to step B1; or a secondary solid strain, corresponding to step B2), and then preparing the secondary strain into a culture strain (step C). Optionally, the culture strain may be subject to further enlarged culture to prepare a culture-enlarged strain (step C1).

A certain volume of culture strains or culture-enlarged strains together with part of the nutrient substrate they carry can be placed in a culture device as a culture mixture for culturing until the mycelia of the strains grow to fill the entire nutrient substrate (step D). Alternatively, secondary liquid strains can be added directly to a culture device pre-filled with nutrient substrate at an inoculum concentration of 1-10% for culturing (a process shown by dashed line in FIG. 1B).

In one embodiment, the culture mixture cultured in step i) of the present invention can be obtained by a method selected from the following:
i-1) culturing a fungus in a slant medium to prepare a mother strain, transferring the mother strain to a liquid medium for continued culture to prepare a secondary liquid strain, and transferring the secondary liquid strain to a new solid medium for continued culture to prepare a liquid culture strain, where the liquid culture strain serves as the fungal strain cultured in step i);
i-2) culturing a fungus in a slant medium to prepare a mother strain, transferring the mother strain to a solid medium for continued culture to prepare a secondary liquid strain, and transferring the secondary liquid strain to a new solid medium for continued culture to prepare a solid culture strain, where the solid culture strain serves as the fungal strain cultured in step i);
i-3) culturing a fungus in a slant medium to prepare a mother strain, transferring the mother strain to a liquid medium for continued culture to prepare a secondary liquid strain, and transferring the secondary liquid strain to a new solid medium for continued culture to prepare a liquid culture strain, where the liquid culture strain is subject to further enlarged culture to prepare a liquid culture-enlarged strain, and the liquid culture-enlarged strain serves as the fungal strain cultured in step i);
i-4) culturing a fungus in a slant medium to prepare a mother strain, transferring the mother strain to a solid medium for continued culture to prepare a secondary liquid strain, and transferring the secondary liquid strain to a new solid medium for continued culture to prepare a solid culture strain, where the solid culture strain is subject to further enlarged culture to prepare a solid culture-enlarged strain, and the solid culture-enlarged strain serves as the fungal strain cultured in step i); and/or
i-5) culturing a fungus in a slant medium to prepare a mother strain, transferring the mother strain to a liquid medium for continued culture to prepare a secondary liquid strain, where the secondary liquid strain serves as the fungal strain cultured in step i).

In one embodiment, in the preceding methods i-1) to i-5), the solid medium is a medium comprising lignin or a derivative thereof as a nutrient substrate. In one embodiment, the nutrient substrate is selected from one or more of the following substances: straw, rice hulls, fuelwood, bark, peanut hulls, corn cobs, twig firewood, wrapper, wood chips and a mixture thereof. In one embodiment, the nutrient substrate consists of 40 wt% straw, 5 wt% rice hulls, 5 wt% wood chips, and 50 wt% water. In one embodiment, the nutrient substrate consists of 45 wt% corn cobs, 5 wt% straw, and 50 wt% water. In one embodiment, the nutrient substrate consists of 45 wt% straw, 5 wt% wood chips, and 50 wt% water.

In one embodiment, in method i-1), the mother strain is obtained by culturing in a PDA slant medium at a temperature of 25-35°C for 5-10 days; the secondary liquid strain is obtained by culturing in a PDA liquid medium in a shaking flask rotating at a speed of 100-200 rpm at a temperature of 25-35°C for 5-10 days; and the secondary liquid strain is transferred to a new solid medium at an inoculum concentration of 1-10% for continued culture to prepare a liquid culture strain.

In one embodiment, in method i-2), the mother strain is obtained by culturing in a PDA slant medium at a temperature of 25-35°C for 5-10 days; the secondary solid strain is obtained by culturing in a solid medium comprising lignin or a derivative thereof as a nutrient substrate at a temperature of 25-35°C for 10-20 days; and the secondary liquid strain is transferred to a new solid medium at an inoculum concentration of 1-10% for continued culture to prepare a solid culture strain.

In one embodiment, in method i-3), the mother strain is obtained by culturing in a PDA slant medium at a temperature of 25-35°C for 5-10 days; the secondary liquid strain is obtained by culturing in a PDA liquid medium in a shaking flask rotating at a speed of 100-200 rpm at a temperature of 25-35°C for 5-10 days; and the secondary liquid strain is transferred to a new solid medium at an inoculum concentration of 1-10% for continued culture to prepare a liquid culture strain; the liquid culture-enlarged strain is obtained by re-transferring the liquid culture strain to a new solid medium at an inoculum concentration of 1-10% for continued culture at 25-35°C for 5-10 days.

In one embodiment, in method i-4), the mother strain is obtained by culturing in a PDA slant medium at a temperature of 25-35°C for 5-10 days; the secondary solid strain is obtained by culturing in a solid medium comprising lignin or a derivative thereof as a nutrient substrate at a temperature of 25-35°C for 10-20 days; and the secondary solid strain is transferred to a new solid medium at an inoculum concentration of 1-10% for continued culture to prepare a solid culture-enlarged strain; the solid culture-enlarged strain is obtained by re-transferring the solid culture strain to a new solid medium at an inoculum concentration of 1-10% for continuing culture at 25-35°C for 5-10 days.

In one embodiment, in method i-5), the culture device in step i) has a solid medium comprising lignin or a derivative thereof as a nutrient substrate; the mother strain is obtained by culturing in a PDA slant medium at a temperature of 25-35°C for 5-10 days; the secondary liquid strain is obtained by culturing in a PDA liquid medium in a shaking flask rotating at a speed of 100-200 rpm at a temperature of 25-35°C for 5-10 days, the secondary liquid strain serves as the fungal strain cultured in step i) at an inoculum concentration of 1-10%.

In one embodiment, in the preceding methods i-1) - i-5), the solid medium is a medium comprising lignin or a derivative thereof as a nutrient substrate. In one embodiment, the nutrient substrate is selected from one or more of the following substances: straw, rice hulls, fuelwood, bark, peanut hulls, corn cobs, twig firewood, wrapper, wood chips and a mixture thereof.

In the present invention, compared with direct use of fungal strains that have not been pre-cultured, using culture strains that have been pre-cultured for culturing has the advantages that the culture strains are rapidly and sufficiently activated, whether in the preparation of liquid strains or the preparation of solid strains, which ensures the high activity of their growth in the nutrient substrate, greatly shortening the growth cycle of the leather-like biocomposite in the later period and facilitating industrialization of the method of the present invention. In addition, the sufficiently activated culture strains are evenly and appropriately distributed in the nutrient substrate, which facilitates uniform growth of mycelium during the formation of the complex, which to a certain extent allows the resulting the leather-like biocomposite prepared by the method of the present invention to have a uniform texture, a soft and fine hand feel, and excellent mechanical properties.

The present invention further comprises the step of post-treating the mycelium-membrane complex obtained after separation. In one embodiment, in step vi), the post-treating involves treating the complex with organic or inorganic reagents, followed by drying it. In one embodiment, the post-treating comprises removing surface proteins of the complex by ethanol and moisturizing the complex by glycerol, and the drying is heat drying, freeze drying or natural air drying. In one embodiment, the post-treating comprises immersing the complex in a mixed solution of ethanol (50%-70%): glycerol of 15-20:1 (v/v) for 8-24 hours, followed by heat treatment at a temperature of 50-80°C for 10-60 minutes.

The fungal strains that can be used in the method of the present invention are selected as fungi whose vegetative part is mycelium, preferably fungi that grow vigorously and whose vegetative part is mycelium, and even more preferably fungi from the class *Basidiomycetes.* In one embodiment, the fungal strain of the present invention is a fungus whose vegetative part is mycelium. In one embodiment, the fungal strain of the present invention is a lower filamentous fungus with mycelium serving as the vegetative part, a common edible fungus with mycelium serving as the vegetative part, or a higher fungus with mycelium serving as the vegetative part. In one embodiment, the fungal strain of the present invention is a fungus from the class *Basidiomycetes.* In one embodiment, the fungal strain of the present invention is one or more selected from the group consisting of: *Agaricus bisporus, Pleurotus ostreatus, Lentinula edodes, Hericium erinaceus, Pleurotus ferulae Lanzi, Flammulina velutipes, Auriclaria polytricha, Ganoderma lucidum, Trametes versicolor,* and *Maitake mushroom.*

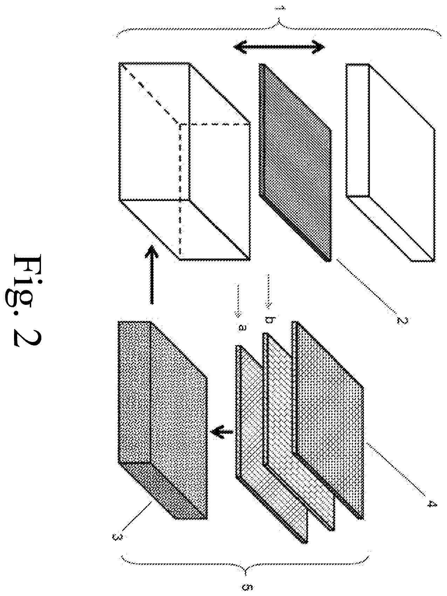

FIG. 2 shows a schematic view of a culture apparatus used for the preparation method of the present invention. As shown in FIG. 2, the culture device 1 of the present invention comprises a receiving portion and a cover capable of sealing the receiving portion. The culture device 1 further comprises a movable plate 2 that is closely fit to the four sides of the receiving portion of the culture device 1, thereby separating the space of the receiving portion into upper and lower portions. Optionally, the culture device 1 further comprises a nutrient substrate 3 and a lattice-like membrane 4.

In one embodiment, the culture device may be a plastic box. The volume of the culture device may be dependent on the desired size of the biocomposite to be produced. In one embodiment, the culture device has a volume of (1-5) meters × (1-5) meters × (0.05-0.1) meters, preferably (1-2.5) meters × (1-2.5) meters × (0.05-0.08) meters. In one embodiment, the culture device is 0.05-0.1 meters, preferably 0.05-0.08 meters in depth.

In the culture device 1, the plate 2 needs to be selected as an unperforated plate and needs to be made of a material that is not easily adhered to and digested by the fungal hyphae. In one embodiment, the unperforated plate is a plastic plate. In one embodiment, the unperforated plate is a glass plate.

The plate 2 in the culture device 1 needs to have a certain weight. A plate with a sufficient weight can provide a better flattening effect, and underweight or overweight of the plate may fail to produce to a densely flattened mycelium. An underweight plate may result in insufficiently dense and compacted mycelium, while an overweight plate may impose increased load on growth of mycelium, thereby decreasing its thickness, even blocking the growth of mycelium to some extent, which can lead to insufficient mycelium per unit area, thus failing to form a dense mycelium that meets the subsequent processing requirements. Selecting the weight of the plate suitable for mycelium growth can be accomplished by the method of the prior art. It has been tested that when the pressure generated by the plate on the mixed culture is 10-20 Pa, preferably 10-15 Pa, the flattening/compacting effect for the mycelium and the effect of maintaining mycelium growth can be balanced. In one embodiment, the pressure generated by the plate on the mixed culture is 10-20 Pa, preferably 10-15 Pa. In one embodiment, the pressure generated by the plate on the mixed culture is 10 Pa, 11 Pa, 12 Pa, 13 Pa, 14 Pa, or 15 Pa.

In one embodiment, a culture mixture comprising a fungal strain and a nutrient substrate 3 is placed in a culture device 1 for culturing the fungal strain until the mycelial of the fungal strain grows and fills the entire nutrient substrate. Subsequently, a plate 2 is flat pressed against the upper surface of the mycelium in one direction and the culture is continued until the mycelium attaching to the side of the plate grows to a thickness of 0.1-5 mm and the formed mycelium completely covers the whole nutrient substrate, forming a mycelial membrane structure on the surface. Subsequently, the plate 2 is removed; a layer of lattice-like membrane 4 is closely attached to the upper surface of the mycelium; and the culture is continued until the mycelium completely penetrates the lattice-like membrane 4 and grows to a height of 0.5-1 cm and distributes evenly, without forming a membrane structure. The plate 2 is flat pressed against the upper surface of the mycelium in one direction and the culture is continued until the mycelium grows to a thickness of 1-5 mm and a mycelium-membrane complex is formed where the mycelium completely covers the lattice-like membrane 4, forming a mycelial membrane structure on the surface thereof. The foregoing steps are repeated at least once, e.g., three times, to obtain a mycelium-membrane complex 5 carrying its underlying nutrient substrate 3 and completely covering three layers of lattice-like membranes 4. After that, the lattice membrane 4b is separated from its underlying mycelium (retaining the second layer of lattice-like membrane as well as the mycelium that covers the membrane and is partially or mostly located thereon) from the lattice-like membrane as shown by "b" in FIG. 2 ("a" represents the first layer of lattice-like membrane; and "b" represents the second layer of lattice-like membrane), thereby separating the mycelium-membrane complex from its underlying nutrient substrate.

In the method of the present invention, it is sufficient to ensure in the separation step that the mycelium-membrane complex is not separated from its underlying nutrient substrate from the first layer of lattice-like membrane. For example, if the foregoing steps are repeated four times, in the case of obtaining a mycelium-membrane complex carrying its underlying nutrient substrate and completely covering four layers of lattice-like membranes 4, it is also possible to optionally discard the bottom two layers of lattice-like membranes and separate the mycelium-membrane complex from its underlying nutrient substrate from the third layer of lattice-like membrane. Thus, in one embodiment, when the foregoing steps are repeated three times, the mycelium-membrane complex is separated from its underlying nutrient substrate from the second layer of lattice-like membrane. In one embodiment, when the foregoing steps are repeated four times, the mycelium-membrane complex is separated from its underlying nutrient substrate from the second or third layer of lattice-like membrane. By discarding the first layer of lattice-like membrane, the obtained mycelium-membrane complex containing the upper layer of lattice-like membrane has a flatter surface, and generally exhibits a fine, uniform and smooth arrangement of mycelium in appearance. The separated lower layer of nutrient substrate comprising the first lattice-like membrane can also be placed again in the culture device for culturing, thereby self-circulating the method of the present invention by repeating it, which greatly reduces the cost of the method of the present invention and is environmentally friendly.

In another aspect, the present invention provides a leather-like biocomposite prepared by the method of the present invention. The leather-like biocomposite prepared by the method of the present invention has excellent appearances including a uniform mycelium arrangement and a soft and fine hand feel, as well as excellent mechanical properties including excellent elongation at break, tear strength, and the like. The elongation at break and breaking strength of the leather-like biocomposite prepared by the method of the present invention can be tested by *Standard Test Method for Breaking Strength and Elongation of Textile Fabrics* (ASTM D5034-09 (2017)). The tear strength of the leather-like biocomposite prepared by the method of the present invention can be tested by the *Test Method for Tearing Strength of Fabrics* (BS EN ISO 13937-2:2000). In one embodiment, the leather-like biocomposite prepared by the method of the present invention has an elongation at break of 20-40 (%), preferably 25-35 (%). In one embodiment, the leather-like biocomposite prepared by the method of the present invention has a breaking strength of 45-60 (lb), preferably 50-57 (lb). In one embodiment, the leather-like biocomposite prepared by the method of the present invention has a tearing strength of 6-25 (N), preferably 9-20 (N).

In yet another aspect, the present invention provides use of the leather-like biocomposite of the invention for preparing a textile product. As the leather-like biocomposite of the present invention is similar to leather materials in appearance, hand feel, texture and mechanical properties, it can be used in the field of textile products as a biocomposite. In one embodiment, the textile product is selected from one or more in the group consisting of: hats, shoes, clothes, and bags. In one embodiment, the textile product is a leather-like substitute.

### Examples

The present invention is further described below in connection with specific examples.

### Example 1- Preparing a leather-like biocomposite of the present invention

### I. Acquisition and identification of a strain

*Trametes versicolor* strains used in this example were commercially available *Trametes versicolor* slant strains purchased from Shenzhen wholesale market for agricultural products. The strain was identified and assessed by referring to [1] TIE Weifang, JIA Junzhong, ZHANG Ye. Cultivation and identification of Pleurotus eryngii strain [J]. China Food Safety Magazine, 2017(27):83-84. DOI:10.16043/j.cnki.cfs.2017.27.065. After the strain was obtained, its genomic DNA was extracted, and then the 18s rDNA fragment was PCR amplified, which was sequenced and identified as *Trametes versicolor* by GeneBank comparison.

### II. Preparing a leather-like biocomposite of the present invention

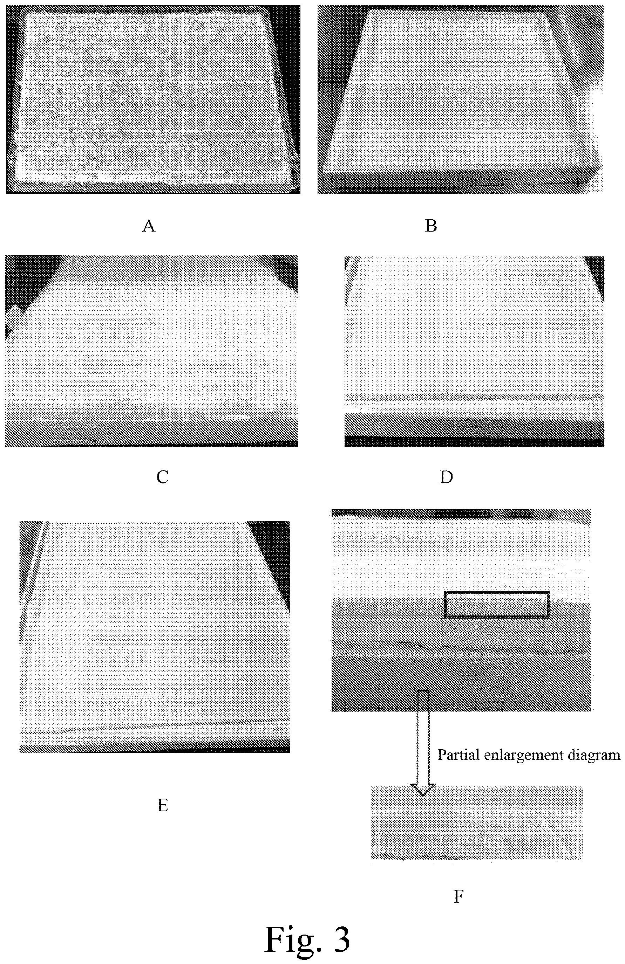

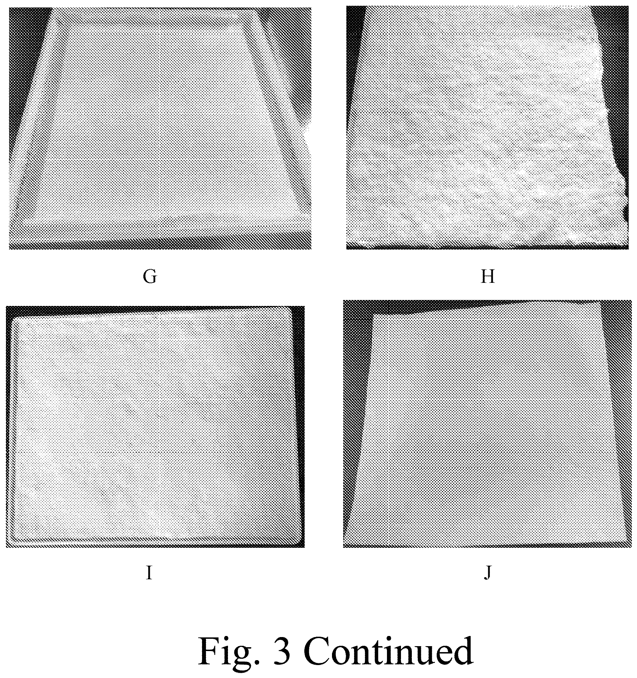

The leather-like biocomposite was prepared by following steps.
1. A piece of *Trametes versicolor* slant strains commercially available (18s identified as *Trametes versicolor*) from the wholesale market for agricultural products was shoveled and inoculated by an inoculation shovel onto the center of PDA slant medium (1 L of potato extract, 20 g of dextrose, 3 g of KH₂PO₄, 1.5 g of MgSO₄·7H₂O, 15 g of agar, 0.05 g of vitamin B1), and placed in an incubator at 25 °C for 7 days such that the mycelium grew all over the slant medium to prepare a mother strain.
2. 5 mL of PDA liquid medium (1 L of potato extract, 20 g of dextrose, 3 g of KH₂PO₄, 1.5 g of MgSO₄·7H₂O, 0.05 g of vitamin B1) was added to the mycelial slant. The upper layer of mycelium was gently scraped into the liquid medium using a sterilized inoculation shovel, and all 5 mL of mycelium-containing media was added into a 250 mL of shake flask containing 45 mL of fresh PDA medium, and incubated at 120 rpm and 25 °C for 5 days until a mycelial ball was produced. Sterilized glass beads (5 mm) were then added to the liquid surface of the medium, and the flask was shaken violently to break up the mycelial ball until there were no visible mycelium blocks, to obtain a secondary liquid strain.
3. The secondary liquid strain was taken at an inoculum concentration of 5% (i.e. 50 mL was taken) and transferred evenly into a culture bag containing 1 kg of sterilized nutrient substrate (40% straw, 5% rice husk, 5% wood chips, and 50%water) for aseptically culturing at 25 °C for 15 days, until the mycelium covered the surface of the nutrient substrate, to obtain a liquid culture strain.
4. The liquid culture strain was taken at an inoculum concentration of 5% (i.e. 500 g was taken) and transferred into a culture bag containing 10 kg of new sterilized nutrient substrate (40% straw, 5% rice husk, 5% wood chips, and 50% water) for aseptically culturing at 25 °C for 10 days, until the mycelium covered the surface of the nutrient substrate, to obtain a liquid culture-enlarged strain.
5. The obtained culture strains were transferred into the bottom of a customized culture device made of plastic with a volume of 1 m × 1 m × 0.05 m at a volume 40% of the volume of the culture device and flat pressed (FIG. 3A), and were kept standstill for culturing in darkness at 25 °C, a carbon dioxide concentration of 5%, an oxygen concentration of 10% and a relative humidity of 75% for 4 days, until the mycelium grew to cover the nutrient substrate (FIG. 3B), The culture was continued and real-time measurements of the mycelium height at the edge of the upper surface of the nutrient substrate were taken, until the mycelium reached a height of 0.6 cm and was evenly distributed (FIG. 3C).
6. The upper surface (FIG. 3E) of the mycelium was flat pressed by a plate (FIG. 3D) in one direction at a pressure of 10 Pa, and was cultured under the above conditions for 4 days until the mycelium attaching to the plate grew to a thickness of 1.5 mm by measurement and the formed mycelium completely covered the entire nutrient substrate, forming a mycelial membrane structure on the surface (FIG. 3F).
7. The plate was uncovered, a cotton gauze with a thickness of 1 mm and an average pore size of 100 µm was used to closely attach to the upper surface of the mycelium (FIG. 3G), and the culture was continued under the above conditions for 4 days until the mycelium at the edge completely penetrated the lattice-like membrane and grew to a height of 0.7 cm by measurement and was evenly distributed, without forming a membrane structure (FIG. 3H).
8. The upper surface of the mycelium was flat pressed once again by a plate in the same direction, the culture was continued under the above conditions for 4 days until the mycelium at the edge grew to a thickness of 1.5 mm by measurement and a mycelium-membrane complex was formed where the mycelium completely covered the lattice-like membrane, forming a mycelial membrane structure on the surface (FIG. 3I).
9. The processes of step 7 to step 8 were repeated once.
10. The second layer of lattice-like membrane was separated from the nutrient substrate attached to the lower part to obtain a monolayer mycelium-membrane complex.
11. The complex was immersed in a mixed solution of ethanol (70%): glycerol of 15:1 (v/v) for 24 hours, followed by heat treatment at 60 °C for 15 minutes to obtain a leather-like biocomposite (FIG. 3J).

### Example 2- Preparing a leather-like biocomposite of the present invention

### I. Acquisition and identification of a strain

*Pleurotus ostreatus* strains used in this example were commercially available *Pleurotus ostreatus* slant strains purchased from Shenzhen wholesale markets for agricultural products. The strain was identified and assessed by using the same method as that in Example 1. After the strain was obtained, its genomic DNA was extracted, and then its 18s rDNA fragment was PCR amplified, which was sequenced and identified as *Pleurotus ostreatus* by GeneBank comparison.

### II. Preparing the leather-like biocomposite of the present invention

The leather-like biocomposite was prepared by the following steps.
1. A piece of *Pleurotus ostreatus* slant strains commercially available (18s identified as *Pleurotus ostreatus*) from the wholesale market for agricultural products was shoveled and inoculated by an inoculation shovel onto the center of PDA slant medium (1 L of potato extract, 20 g of dextrose, 3 g of KH₂PO₄, 1.5 g of MgSO₄·7H₂O, 15 g of agar, 0.05 g of vitamin B1), and placed in an incubator at 28 °C for 7 days such that the mycelium grew all over the slant medium to prepare the mother strains.
2. A piece of mycelium was shoveled by a sterilized inoculation shovel and transferred into a culture bag containing 500 g of new sterilized nutrient substrate (45% corn husk, 5% straw, and 50% water) for culturing at 28 °C for 18 days, until the mycelium covered the surface of the nutrient substrate, to obtain a secondary solid strain.
3. The secondary liquid strain was taken at an inoculum concentration of 5% (i.e. 500 g was taken) and mixed evenly in a culture bag containing 10 kg of fresh sterilized nutrient substrate (45% corn husk, 5% straw, and 50% water) for aseptically culturing at 28 °C for 10 days, until the mycelium covered the surface of the nutrient substrate, to obtain a culture strain.
4. The obtained culture strains were transferred to the bottom of a customized culture device made of plastic with a volume of 1.5 m × 1.2 m × 0.06 m at a volume 55% of the volume of the culture device and flat pressed, and were kept standstill for culturing in darkness at 28 °C, a carbon dioxide concentration of 5%, an oxygen concentration of 12% and a relative humidity of 80% for 5 days. The mycelium at the edge of the upper surface of the nutrient substrate was then measured to 0.5 cm in height and was evenly distributed.
5. The upper surface of the mycelium was flat pressed by a plate in one direction at a pressure of 14 Pa, and was cultured under the above conditions for 5 days until the mycelium attaching to the plate grew to a thickness of 1 mm by measurement and the formed mycelium completely covered the entire nutrient substrate, forming a mycelial membrane structure on the surface.
6. The plate was uncovered, a cotton gauze with a thickness of 0.5 mm and an average pore size of 80 µm was used to closely attach to the upper surface of the mycelium, and the culture was continued under the above conditions for 5 days until the mycelium at the edge completely penetrated the lattice-like membrane and grew to a height of 0.5 cm by measurement and was evenly distributed, without forming a membrane structure.
7. The upper surface of the mycelium was flat pressed again by a plate in the same direction and the culture was continued under the above conditions for 5 days until the mycelium at the edge grew to a thickness of 1 mm by measurement and a mycelium-membrane complex was formed where the mycelium completely covered the lattice-like membrane, forming a mycelial membrane structure on the surface.
8. The processes of step 6 to step 7 were repeated twice.
9. The second layer of lattice-like membrane was separated from the nutrient substrate attached to its lower part to obtain a bilayer mycelium-membrane complex.
10. The complex was immersed in a mixed solution of ethanol (70%): glycerol of 19:1 (v/v) for 24 hours, followed by heat treatment at 80 °C for 10 minutes to obtain a leather-like biocomposite.

The pictures obtained from the various steps in this example are similar to those in Example 1 and will not be shown again.

### Example 3- Preparing a leather-like biocomposite of the present invention

### I. Acquisition and identification of a strain

*Lentinula edodes* strains used in this example were commercially available *Lentinula edodes* slant strains purchased from Shenzhen wholesale markets for agricultural products. The strain was identified and assessed by using the same method as that in Example 1. After the strain was obtained, its genomic DNA was extracted, then its 18s rDNA fragment was PCR amplified, which was sequenced and identified as *Lentinula edodes* by GeneBank comparison.

### II. Preparing the leather-like biocomposite of the present invention

The leather-like biocomposite was prepared by the following steps.
1. A piece of *Lentinula edodes* slant strains (18s identified as *Lentinula edodes*) commercially available from the wholesale market for agricultural products was shoveled and transferred by an inoculation shovel onto the center of PDA slant medium (1 L of potato extract, 20 g of dextrose, 3 g of KH₂PO₄, 1.5 g of MgSO₄·7H₂O, 15 g of agar, 0.05 g of vitamin B1), and placed in an incubator at 26 °C for 6 days such that the mycelium grew all over the slant medium to prepare the mother strains.
2. 5 mL of PDA liquid medium (1 L of potato extract, 20 g of dextrose, 3 g of KH₂PO₄, 1.5 g of MgSO₄·7H₂O, 0.05 g of vitamin B1) was added to the mycelial slant. The upper layer of mycelium was gently scraped into the liquid medium using a sterilized inoculation shovel, and all 5 mL of mycelium-containing medium was added into a 250 mL of shake flask containing 45 mL of fresh PDA medium, and incubated at 120 rpm at 26 °C for 5 days until a mycelial ball was produced. Sterilized glass beads (5 mm) were then added to the liquid surface of the medium, the flask was shaken violently to break up the mycelia ball until there were no visible mycelium blocks, to obtain a secondary liquid strain.
3. 10 kg of sterilized nutrient substrate (45% straw, 5% wood chips, and 50% water) were transferred to the bottom of a customized culture device made of plastic with a volume of 1.1 m × 1.1 m × 0.05 m at a volume 50% of the volume of the culture device and compacted and flat pressed. The secondary liquid strain was added dropwise to the nutrient substrate evenly at an inoculum concentration of 10% and kept standstill for culturing in darkness at 26 °C, a carbon dioxide concentration of 6%, an oxygen concentration of 8% and a relative humidity of 70% in darkness for 20 days. The mycelium at the edge of the upper surface of the nutrient substrate was measured to 0.7 cm in height and was evenly distributed.
4. The upper surface of the mycelium was flat pressed by a plate in one direction at a pressure of 12 Pa, and was cultured under the above conditions for 4 days until the mycelium attaching to the plate grew to a thickness of 2 mm by measurement and the formed mycelium completely covered the entire nutrient substrate, forming a mycelial membrane structure formed on the surface.
5. The plate was uncovered, a pure cotton non-woven fabric with a thickness of 1 mm and an average pore size of 120 µm was used to closely attach to the upper surface of the mycelium, and the culture was continued under the above conditions for 4 days until the mycelium at the edge completely penetrated the lattice-like membrane and grew to a height of 0.8 cm by measurement and was evenly distributed, without forming a membrane structure.
6. The upper surface of the mycelium was flat pressed again by a plate in the same direction and the culture was continued under the above conditions for 4 days until the mycelium at the edge grew to a thickness of 2 mm by measurement and a mycelium-membrane complex was formed where the mycelium completely covered the lattice-like membrane, forming a mycelial membrane structure on its surface.
7. The processes of step 5 to step 6 were repeated three times.
8. the second layer of lattice-like membrane was separated from the nutrient substrate attached to its lower part to obtain a trilayer mycelium-membrane complex;
9. the complex was immersed in a mixed solution of ethanol (50%): glycerol of 18:1 (v/v) for 24 hours, followed by heat treatment at 70 °C for 15 minutes to obtain a leather-like biocomposite.

The pictures obtained from the various steps in this example are similar to those in Example 1 and will not be shown repeatedly.

### Example 4-Cmparative evaluation of the mycelial membrane structure of the present invention with a non-mycelial membrane structure.

To evaluate the effect on the formation of the mycelial membrane structure and the appearance of the mycelium-membrane complex by flat pressing using an unperforated plate, a monolayer mycelium-membrane complex was prepared using the method described in Example 1, except that only half of the mycelium was flat pressed by the unperforated plate during its growth, while the other half did not undergo such operation.

FIG. 4 shows physical comparison of the mycelial membrane structure obtained by this example with a non-mycelial membrane structure. As shown in FIG. 4, since the mycelium in the lower part of FIG. 4 has been treated by the method of the present invention during culturing (by flat pressing using an unperforated plate), this part of mycelium has good integrity with a flat structure and a smooth, leather-like hand feel, forming a mycelial membrane structure (see the partial enlargement diagram in the lower right of FIG. 4). On contrary, the mycelium in the upper part of FIG. 4 did not undergo the operation of flat pressing using an unperforated plate during culturing. The mycelium cultured in this part grew freely towards the vertical plane, forming individual hyphae with an overall appearance of a fluffy, non-membranous structure. Enlargement of this partial structure shows that these hyphae are densely distributed without an integral structure (see the partial enlargement view at the lower left part of FIG. 4).

### Example 5 - Comparison of the structures of mycelium layers obtained by plate pressing of the present invention and by rolling as a control

The strains and method of Example 1 of the present invention were used for the present experiment, except that a plate was not used in steps 6 and 8 to apply pressure to the upper surface of the mycelium. Instead, a roller with a smooth surface was used to apply pressure to the upper surface of the mycelium by means of rolling. The experiments were carried out in parallel in three groups and the pressure applied was adjusted with reference to the plate pressing. The group 1 was applied a single light rolling; the group 2 was applied two times of moderate rolling; and the group 3 applied multiple times of heavy rolling. In addition, the experiment of Example 1 was repeated as a control, which was numbered as group 4.

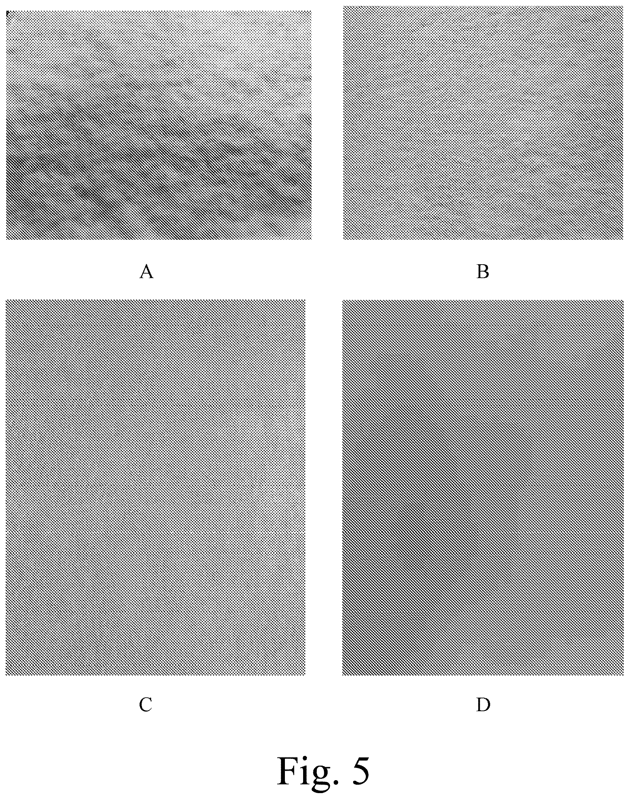

FIG. 5 shows physical diagrams of the mycelium layer structures obtained from group 1 (FIG. 5A), group 2 (FIG. 5B), group 3 (FIG. 5C) and group 4 (FIG. 5D).

As shown in FIG. 5A, after the single light rolling, the final mycelial membrane was less likely to form a uniform plane, exhibiting apparently uneven undulations. As shown in FIG. 5B, after the two times of moderate rolling, the mycelium layer still exhibited apparent unevenness. As shown in FIG. 5C, after multiple times of heavy rolling, the mycelium layer was able to be flat pressed but with irregular hyphae, showing a fluffing and pilling appearance. As shown in FIG. 5D, after plate pressing of the present invention and pressing the plate along the same direction twice, the final mycelium layer resulted in a glossy, dense and flawless plane, with a mycelial membrane structure formed on the surface. Additionally, it has been found in experiments that the mycelial membrane would not be formed by further culture after rolling. Similarly, if the plate was removed after plate pressing, mycelial membrane was not formed by further culturing. Continuous plate pressing during culture facilitated the formation of mycelial membrane. The above results suggest that the plate pressing method is very important for the final formation of the mycelial membrane structure that presents a smooth and dense surface in appearance, as well as for finally obtaining the leather-like biocomposite that has a flat appearance and a fine hand feel, as compared to the rolling method.

### Example 6 - Performance testing of the mycelium-membrane complex covering a monolayer lattice-like membrane of the present invention

To evaluate the mechanical properties of the mycelium-membrane complex obtained by the method of the present invention, the monolayer mycelium-membrane complex prepared using the method described in Example 1 was tested for mechanical properties including elongation at break, breaking strength, and tear strength.

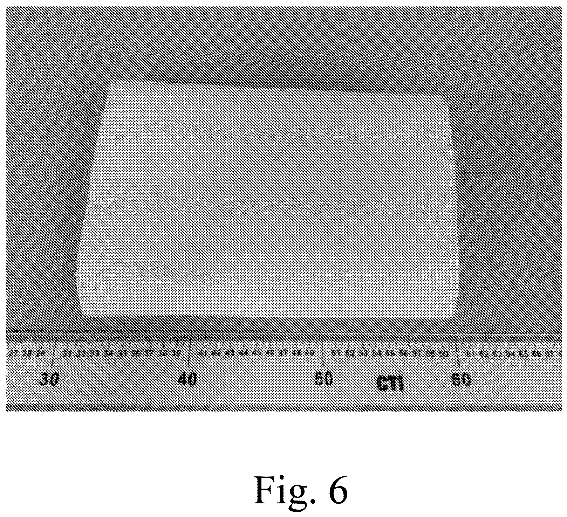

The test was commissioned to the Center Testing International (CTI) located in Bao'an District, Shenzhen, Guangdong Province, with the test report numbering A2210375490101C. FIG. 6 shows a physical diagram of a leather-like biocomposite obtained by the method of examples of the present invention after post-treating, which was sent to CTI for testing mechanical properties. The elongation at break and breaking strength of the leather-like biocomposite prepared by Example 1 can be tested by *Standard Test Method for Breaking Strength and Elongation of Textile Fabrics* (ASTM D5034-09 (2017)). The tear strength of the leather-like biocomposite prepared by Example 1 can be tested by the *Test Method for Tearing Strength of Fabrics* (BS EN ISO 13937-2:2000).

The testing results of breaking strength are shown in table 1 below.

| Testing item | Testing result | Standard requirements |
|---|---|---|
| | 001 | |
| Length direction | 19.6N* | -- |
| Width direction | 9.6N | -- |

| | | |
|---|---|---|
| Note: *= The tearing direction is vertical to the sample cut. | | |

The testing results of tear strength are shown in table 2 below.

| Testing item | Testing result | Standard requirements |
|---|---|---|
| | 001 | |
| Length direction | 57.4lb | -- |
| Width direction | 50.5lb | -- |

The test results of elongation at break are shown in table 3 below.

| Testing item | Testing result | Standard requirements |
|---|---|---|
| | 001 | |
| Length direction | 26.6% | -- |
| Width direction | 35.2% | -- |

Based on the above test results, it can be seen that the complex obtained by using the method of the present invention, in particular the operation of flat pressing by an unperforated plate in a random direction, shows good mechanical properties in different directions and excellent overall mechanical properties.

### Example 7 - Comparison of mechanical properties of composites obtained by plate pressing of the present invention and by rolling as a control

The mechanical properties of the monolayer mycelium-membrane complexes obtained by the methods of group 3 and group 4 in Example 5 were tested using the test method in Example 6. The tested indicators include breaking strength and tear strength.

The test results of breaking strength and tear strength are shown in table 4 below.

| | Rolling (group 3) | Plate pressing (group 4) |
|---|---|---|
| Breaking strength (lb) | Longitudinal: 34 | Vertical: 57 |
| | Horizontal: 15 | Horizontal: 51 |
| Tear strength(N) | Vertical: 8 | Longitudinal: 20 |
| | Horizontal: 3 | Horizontal: 10 |

The result shows that the test results for the breaking strength and tear strength of the monolayer mycelium-membrane complex obtained by the plate pressing method of the present invention increased by at least a 1-fold in both the longitudinal and horizontal directions, exhibiting a significant improvement in the overall mechanical properties, as compared to the rolling method (even with multiple times of heavy rolling). This result again indicates the importance of applying pressure through plate pressing of the present invention and pressing the plate along the same direction twice to ultimately improve the mechanical properties of the mycelium-membrane complex.

### Example 8 - Post-treating of the mycelium-membrane complex of the present invention

The obtained mycelium-membrane complex was post-treated by using the test method of Example 1. Specifically, the complex was immersed in a mixed solution of ethanol (70%): glycerol of 15:1 (v/v) for 24 hours, followed by heat treatment at 60 °C for 15 minutes to obtain a leather-like biocomposite.

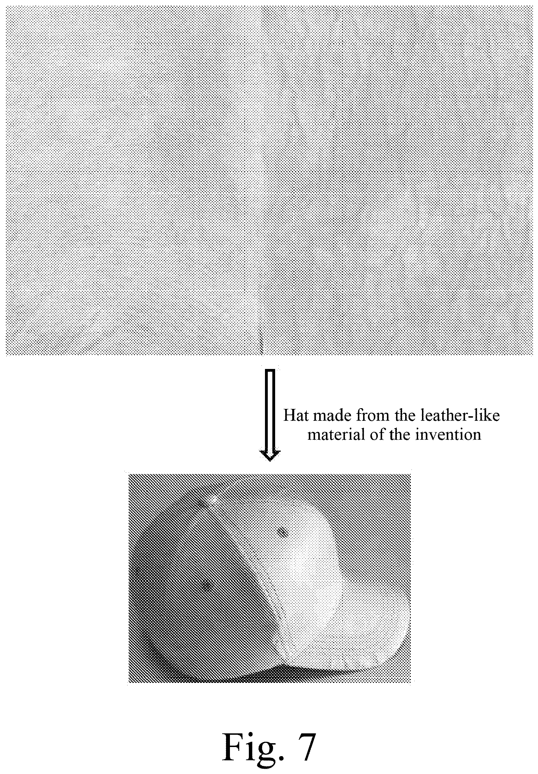

FIG. 7 shows a physical diagram of a leather-like biocomposite obtained by the method of Example 1 of the present invention after post-treating, and a hat made therefrom. As seen from the physical composite diagrams of FIG. 5 and FIG. 7, the composite prepared by the method of the present invention has a flat appearance, dense structure, good integrity, and a fine and smooth hand feel, which can completely replace the animal-derived leather to produce textile products.

The above examples are merely examples for the sake of clarity and are not a limitation on the embodiments. For those of ordinary skill in the art, other changes or variations made on the basis of the above description are still within the scope of the present invention.

## Claims

1. A method for preparing a leather-like biocomposite, comprising the following steps:
i). placing a culture mixture comprising a fungal strain and a nutrient substrate in a culture device and culturing the fungal strain until the mycelium of the fungal strain grows and fills the entire nutrient substrate and grows to a height of 0.5-1 cm and distributes evenly;
ii). flat pressing an unperforated plate against the upper surface of the mycelium in one direction and continuing culturing until the mycelium attaching to the plate grows to a thickness of 0.1-5 mm and the formed mycelium completely covers the entire nutrient substrate, forming a mycelial membrane structure on the surface thereof;
iii). removing the unperforated plate, closely attaching a lattice-like membrane on the upper surface of the mycelium and continuing culturing until the mycelium completely penetrates the lattice-like membrane and grows to a height of 0.5-1 cm and distributes evenly, without forming a mycelial membrane structure;
iv). flat pressing the unperforated plate against the upper surface of the mycelium in one direction and continuing culturing until the mycelium grows to a thickness of 1-5 mm and a mycelium-membrane complex is formed where the mycelium completely covers the lattice-like membrane, forming a mycelial membrane structure again on the surface;
v). repeating the preceding steps iii) and iv) at least once; and
vi). separating the complex from the nutrient substrate and post-treating the complex to obtain a leather-like biocomposite.

2. The method according to claim 1, wherein in the steps ii) and iv), the unperforated plate is flat pressed against the upper surface of the mycelium in the same direction.

3. The method according to claim 1 or 2, further comprising: in the step v), repeating the preceding steps iii) and iv) two or more times to form a complex comprising a multilayer structure; preferably, repeating the steps ii)-v) 2 to 5 times;
in the step vi), separating the complex from the nutrient substrate from at least the second layer of the lattice-like membrane; and/or
placing the separated nutrient substrate in the step vi) in a culture device again for culturing, and then repeating steps ii)-v) for circulated preparation of the leather-like biocomposite.

4. The method according to any one of claims 1-5, wherein in the step i), the fungal strain is cultured under the following conditions: a nutrient substrate comprising lignin or a derivative thereof as the nutrient substrate, a volume ratio of the culturing mixture in the culture device of up to 60%, a culture duration of 3-7 days, a temperature of 25-35 °C, standing in darkness, a concentration of carbon dioxide of 3-7%, a concentration of oxygen ≤20%, and a relative humidity ≥ 40%;wherein preferably, the nutrient substrate is selected from one or more of the following substances: straw, rice hulls, fuelwood, bark, peanut hulls, corn cobs, twig firewood, wrapper, wood chips and a mixture thereof.

5. The method according to any one of claims 1-4, wherein
in the step ii), the unperforated plate is flat pressed against the upper surface of the mycelium and kept standstill for continuous culturing in darkness for 3-7 days at a temperature of 25-35°C, a carbon dioxide concentration of 3-7%, an oxygen concentration ≤20%, and a relative humidity ≥40%;
in the step iii), the lattice-like membrane is closely attached to the upper surface of the mycelium and kept standstill for continuous culturing in darkness for 3-7 days at a temperature of 25-35°C, a carbon dioxide concentration of 3-7%, an oxygen concentration ≤20%, and a relative humidity ≥40% and/ or
in the step iv), the unperforated plate is flat pressed against the upper surface of the mycelium and kept standstill for continuous culturing in darkness for 3-7 days at a temperature of 25-35 °C, a carbon dioxide concentration of 3-7%, an oxygen concentration ≤20%, and a relative humidity ≥40%.

6. The method according to any one of claims 1-5, wherein the culture mixture cultured in the step i) is obtained by a method selected from the followings:
i-1) culturing the fungus in a slant medium to prepare a mother strain, transferring the mother strain to a liquid medium for continued culture to prepare a secondary liquid strain, and transferring the secondary liquid strain to a new solid medium for continued culture to prepare a liquid culture strain, wherein the liquid culture strain serves as the fungal strain cultured in the step i);
preferably, in method i-1), the mother strain is obtained by culturing in a PDA slant medium at a temperature of 25-35 °C for 5-10 days; the secondary liquid strain is obtained by culturing in a PDA liquid medium in a shaking flask rotating at a speed of 100-200 rpm at a temperature of 25-35 °C for 5-10 days; and the secondary liquid strain is transferred to a new solid medium at an inoculum concentration of 1-10% for continued culture to prepare a liquid culture strain;
i-2) culturing the fungus in a slant medium to prepare a mother strain, transferring the mother strain to a solid medium for continued culture to prepare a secondary liquid strain, and transferring the secondary liquid strain to a new solid medium for continued culture to prepare a solid culture strain, wherein the solid culture strain serves as the fungal strain cultured in the step i);
preferably, in method i-2), the mother strain is obtained by culturing in a PDA slant medium at a temperature of 25-35 °C for 5-10 days; the secondary solid strain is obtained by culturing in a solid medium comprising lignin or a derivative thereof as a nutrient substrate at a temperature of 25-35 °C for 10-20 days; and the secondary liquid strain is transferred to a new solid medium at an inoculum concentration of 1-10% for continued culture to prepare a solid culture strain;
i-3) culturing the fungus in a slant medium to prepare a mother strain, transferring the mother strain to a liquid medium for continued culture to prepare a secondary liquid strain, and transferring the secondary liquid strain to a new solid medium for continued culture to prepare a liquid culture strain, and the liquid culture strain is further enlarged cultured to prepare a liquid culture-enlarged strain, wherein the liquid culture-enlarged strain serves as the fungal strain cultured in the step i);
preferably, in method i-3), the mother strain is obtained by culturing in a PDA slant medium at a temperature of 25-35 °C for 5-10 days; the secondary liquid strain is obtained by culturing in a PDA liquid medium in a shaking flask rotating at a speed of 100-200 rpm at a temperature of 25-35°C for 5-10 days; and the secondary liquid strain is transferred to a new solid medium at an inoculum concentration of 1-10% for continued culture to prepare a liquid culture strain; the liquid culture-enlarged strain is obtained by re-transferring the liquid culture strain to a new solid medium at an inoculum concentration of 1-10% for continued culture at 25-35 °C for 5-10 days;
i-4) culturing the fungus in a slant medium to prepare a mother strain, transferring the mother strain to a solid medium for continued culture to prepare a secondary liquid strain, and transferring the secondary liquid strain to a new solid medium for continued culture to prepare a solid culture strain, and the solid culture strain is further enlarged cultured to prepare a solid culture-enlarged strain, wherein the solid culture-enlarged strain serves as the fungal strain cultured in the step i);
preferably, in method i-4), the mother strain is obtained by culturing in a PDA slant medium at a temperature of 25-35°C for 5-10 days; the secondary solid strain is obtained by culturing in a solid medium comprising lignin or a derivative thereof as a nutrient substrate at a temperature of 25-35°C for 10-20 days; and the secondary solid strain is transferred to a new solid medium at an inoculum concentration of 1-10% for continued culture to prepare a solid culture-enlarged strain; the solid culture-enlarged strain is obtained by re-transferring the solid culture strain to a new solid medium at an inoculum concentration of 1-10% for continuing culture at 25-35 °C for 5-10 days; and/or
i-5) culturing the fungus in a slant medium to prepare a mother strain, transferring the mother strain to a liquid medium for continued culture to prepare a secondary liquid strain, wherein the secondary liquid strain serves as the fungal strain cultured in the step i);
preferably, in method i-5), the culture device in the step i) has a solid medium comprising lignin or a derivative thereof as a nutrient substrate; the mother strain is obtained by culturing in a PDA slant medium at a temperature of 25-35°C for 5-10 days; the secondary liquid strain is obtained by culturing in a PDA liquid medium in a shaking flask rotating at a speed of 100-200 rpm at a temperature of 25-35 °C for 5-10 days, wherein the secondary liquid strain serves as the fungal strain cultured in the step i) at an inoculum concentration of 1-10%.

7. The method according to any one of claims 1-6, wherein in the step vi), the post-treating comprises treating the complex with an organic or inorganic reagent followed by drying the complex;
preferably, the post-treating comprises removing surface proteins of the complex by ethanol and moisturizing the complex by glycerol, and the drying is heat drying, freeze drying or natural air drying;
more preferably, the post-treating comprises immersing the complex in a mixed solution of ethanol (50%-70%): glycerol of 15-20:1 (v/v) for 8-24 hours, followed by heat treatment at a temperature of 50-80 °C for 10-60 minutes.

8. The method according to any one of claims 1-7, wherein the lattice-like membrane has a thickness of 0.1 m-5 mm and an average pore size of 1 µm-5 mm; preferably, the thickness is 0.5 mm and the average pore size is 0.1 mm-5 mm; and/or wherein the lattice-like membrane is one or more selected from: a gauze, a woven nylon fabric, and a natural fiber fabric.

9. The method according to any one of claims 1-8, wherein the fungus is a fungus whose vegetative part is mycelium; preferably, the fungus is a lower filamentous fungus, a common edible fungus, or a higher fungus; more preferably, the fungus is a fungus from the class *Basidiomycetes;* more preferably, the fungus is selected from one or more in the group consisting of: *Agaricus bisporus, Pleurotus ostreatus, Lentinula edodes, Hericium erinaceus, Pleurotus ferulae Lanzi, Flammulina velutipes, Auriclaria polytricha, Ganoderma lucidum, Trametes versicolor,* and *Maitake mushroom.*

10. A leather-like biocomposite prepared by the method according to any one of claims 1-11; wherein preferably, the leather-like biocomposite has an elongation at break of 20-40 (%), preferably 25-35 (%), a breaking strength of 45-60 (lb), preferably 50-57 (lb), and a tear strength of 6-25 (N), preferably 9-20 (N).

11. Use of the leather-like biocomposite prepared by the method according to any one of claims 1-9 or the leather-like biocomposite according to claim 10 for preparing a textile product; wherein preferably, the textile product is selected from one or more in the group consisting of: hats, clothes, and bags.
